# EUROPEAN PATENT SPECIFICATION

(11) **EP 1 448 244 B1**
(45) Date of publication and mention of the grant of the patent: **28.02.2007**
(21) Application number: 02791143.7
(22) Date of filing: 26.11.2002
(51) Int. Cl.: A61L 15/60

(54) **NEW ABSORBENT PRODUCT**
NEUES SAUGFÄHIGES PRODUKT
PRODUIT ABSORBANT

(30) Priority: 27.11.2001 SE 0103961
(43) Date of publication of application: 25.08.2004
(73) Proprietor: SCA Hygiene Products AB, 405 03 Göteborg (SE)
(72) Inventor: FALK, Torgny, S-422 41 Hisings-Backa (SE)
(74) Representative: Bergstrand, Mikael Gudmundsson
(86) International application number: PCT/SE2002/002171
(87) International publication number: WO 2003/045453

(56) References cited:
- WO-A1-94/15651
- WO-A1-96/15154
- US-A- 4 654 039
- US-A- 5 241 009
- US-A- 5 462 972
- US-A- 5 985 432

## Description

### Technical field

The invention refers to an absorbent product, such as a panty liner, a sanitary towel, or an incontinence protection for absorption of blood or menstrual fluid, intended to be worn in the crotch part of an undergarment, comprising a fluid permeable surface layer facing the wearer during use, a fluid impermeable backing layer facing away from the wearer during use, and an absorbent body comprising one or more material layers, positioned between the backing layer and the surface layer, whereby the absorbent body comprises super-absorbent polymers (SAP), and that the SAP-material shows a contact angle at its surface which is smaller than 60°, as measured with defibrinated sheep blood.

### Technical background

In order to absorb fluid in absorbent products, so-called super-absorbents (SAP) are often used. These have a capability to absorb fluid in a quantity several times their own weight, and for this reason they have shown to be very suitable for use in products of this kind. Large amounts of liquid may quickly and efficiently be absorbed. The absorbent core of the products may be relatively small, which makes the product less bulky at use and at package.

Normally, SAP is added to the rest of the core structure in the form of polymer particles. These particles are built up of super-absorbent polymer chains forming a network, which is held together by cross-linkings. In order to obtain these cross-linkings cross-linking agents are used.

For example, DE-A-19941423 discloses a SAP-polymer and its production, which comprises (a) 55-99.9 % (by weight) of an ethylene-containing polymerisable monomer, (b) 0-40 % (by weight) of an with (a) co-polymerisable monomer, (c) 0.01-5.0, preferably 0.1-2.0, % (by weight) of a cross-linking agent, and (d) 0-30 % (by weight) of a water-soluble polymer.

Most available absorbent SAP-products are adapted for urine absorption. However, the invention is primarily conferred to absorption of blood, which partly provides other problems compared to urine absorption. In comparison to blood, urine has a much higher water content. Conventional SAP:s are adapted for absorption of water.

Blood is a fluid having a high viscosity. To about 45 % blood comprises of blood cells, and to about 55 % of blood plasma. The blood plasma comprises of salts, water and proteins (e.g. albumin, IgG and fibrinogen). The proteins add up to about 7-8 % of the blood plasma. The amount of protein in blood is in the order of 10¹⁷/ml blood, and the amount of blood cells is in the order of 10⁹/ml blood.

At absorption of blood to a SAP-particle, the proteins will interact to a much greater extent with the surface of the SAP-particles, compared to the blood cells. SAP-particles are primarily adapted for water absorption. Therefore, it is vital to design the surface of the SAP-particles in such a way that the transport of water molecules into the particles is favored. By applying a hydrophilic surface to the SAP-particles, the tendency of the proteins to interact with the surface of the particles will decrease. Tests (Nadarajah et al., "Modeling the Dynamics of Proteins Adsorption to Surfaces", from ACS Symposium Series 602, Proteins at Interfaces II - Fundamentals and Applications (Horbett and Brash, 1995): chapter 13) have shown that a hydrophilic surface results in a lower protein interaction. However, the water molecules are polar, and will therefore interact to a high degree with a hydrophilic surface. The same is applicable for salts, which in water solution occur in ionic form, which also will be attracted by the surface of the SAP-particles. In this way, the transport of water and salt to the surface of the SAP-particles, and thereby also the possibility for them to be absorbed into the SAP-particles, will increase, at the expense of the protein assembly at the surface, if the surface of the SAP-particles is hydrophilic. Moreover, a large difference in ion concentration within and outside a SAP-particle may result in a high osmotic pressure, which contributes to a strong absorption capacity.

The degree of cross-linking determines the elasticity of the material, as well as its absorbent properties. A higher degree of cross-linking results in a more brittle material, but also a higher initial ability to absorb fluid, i.e. the SAP becomes quick, but obtains a lower total capacity to absorb a large volume, i.e. a lower total capacity. A low degree of cross-linking gives a viscous, expandable material, which has the capacity to absorb a large volume of liquid. However, a low degree of cross-linking may result in a relatively slow absorption. A reason for this is that the diffusion constant increases and the swelling is faster (at a higher cross-linking degree), but the total capacity decreases ("Modem superabsorbent polymer technology", Buchholz and Graham, Wiley-VCH, 1998). A balance between these two extremes is therefore desirable.

A high degree of cross-linking also results in a high gel-strength. The gel-strength is a measure of the ability of the SAP-gel to withstand pressure without losing fluid. A SAP having a high gel-strength thus has a high ability to keep fluid when it is subjected to load, such as when the wearer of an absorbent product sits down or lies in a way that the article, and the SAP-particle within it, is pressed together.

US-A-5241009 discloses an absorbent product, which may be used for absorption of blood. In this text, a cross-linking degree of about 0.1-2.0 % is recommended at blood absorption.

WO94/15651 discloses a SAP being specially adapted for absorption under pressure, which comprises 0.1-5 % (weight) of a cross-linking agent.

US-A-5985432 discloses a SAP having a cross-linking degree of 0.001 to 5 % mole. The absorbent material comprises a polyether or/and a polycation, which is bound to the absorbing polymer, resulting in a contact angle for blood of 0 to 40 °.

Many of the SAP-containing absorbent products, which are used today for absorption of blood and menstrual fluids, are based on polyacrylic acid, which is a standard SAP-material. Other base materials for SAP:s exist, such as for example CMC (cellulose) or starch.

Thus, today some products for blood absorption exist. However, these are to a great extent also adapted for urine absorption. Therefore, it is of interest to improve the absorption characteristics for the existing products, not the least by optimisation of the chemical construction of the SAP-material, in order to provide a SAP-material which has improved absorption characteristics for blood.

The object of the invention is to provide an absorbent product, which solves the problems stated above, thereby providing a product having improved absorption properties for blood.

### Summary of the invention

The inventor of the present invention has surprisingly found that by using a SAP-material having a gel-strength which exceeds 18000 Pa, and having a suitable wettability on the surface of the particles, a strongly improved absorption capacity for blood is obtained, compared to the materials used today.

Accordingly, in a first embodiment the invention relates to an absorbent product, such as a panty liner, a sanitary towel, or an incontinence protection for absorption of blood or menstrual fluid, intended to be worn in the crotch part of an undergarment, comprising a fluid permeable surface layer facing the wearer during use, a fluid impermeable backing layer facing away from the wearer during use, and an absorbent body comprising one or more material layers, positioned between the surface layer and the backing layer, whereby the absorbent body comprises super-absorbent polymers (SAP), and that the SAP-material shows a contact angle at its surface which is smaller than 60°, as measured by fresh, sterile and defibrinated sheep blood, characterized by that the SAP-material shows a gel-strength that exceeds 18000 Pa.

Furthermore, the inventor has found that a SAP-material suitable for use in the invention has a cross-linking degree in the interval from 3.0-7.0 % mole.

That SAP-particles having a cross-linking degree within this interval works well for absorption of blood is clearly surprising, since it has been thought that a too low expandability is obtained with a cross-linking degree as high as this. However, the inventor has shown that this low expandability is compensated by that the gel-strength results in a high initial absorption capacity for blood, and that this in combination with a suitably chosen wettability on the surface of the SAP-material, gives SAP-material that is especially suited for absorption of blood or blood-containing fluids.

By choosing a gel-strength and/or a cross-linking degree within this interval and a contact angle smaller than 60°, and preferably smaller than 40°, a balance between hydrophilicity on the surface of the SAP and absorption capacity within the SAP is achieved.

Moreover, the SAP that is used in the invention do not only have a high degree of cross-linking, but also some degree of non-cross-linked, so-called free polymers in its structure. These free polymers give no contribution to the degree of cross-linking, but they improve the gel-strength. Hereby, a high osmotic pressure is achieved and thereby a high initial absorption capacity. The free polymers give an "imaginary cross-linking", i.e. they strengthen the osmotic pressure without making the gel harder and less swellable. According to a preferred embodiment, the SAP of the invention thus has a gel-strength above 18000 Pa, more preferably above 25000 Pa, and most preferably above 30000 Pa.

In one embodiment the invention relates to an absorbent product, containing SAP-material as above, which has an absorption capacity for blood higher than 6g blood/g SAP, preferably higher than 10 g blood/g SAP, most preferably higher than 13 g blood/g SAP, whereby the given absorption capacity values are measured at absorption under load (AUL) of 2 kPa. Moreover, it is possible to mix various types of SAP, having different absorption capacities, in an absorbent product.

In yet another embodiment, the invention relates to an absorbent product, in which the surface of the SAP-particles is treated with a surface modifier, such as AlCl₃ and/or Al₂(SO₄)₃. Hereby, the SAP-particles obtain desired surface properties, which in turn facilitate a good absorption of blood.

### Short description of the drawings

Figure 1 shows an example of a sanitary towel according to the invention.

Figure 2 shows a graph describing the absorption capacity as a function of the degree of cross-linking for different liquids and SAP-materials.

Figure 3 shows a graph describing the absorption capacity as a function of the SAP-gel-strength for different liquids and SAP-materials.

### Detailed description of the invention

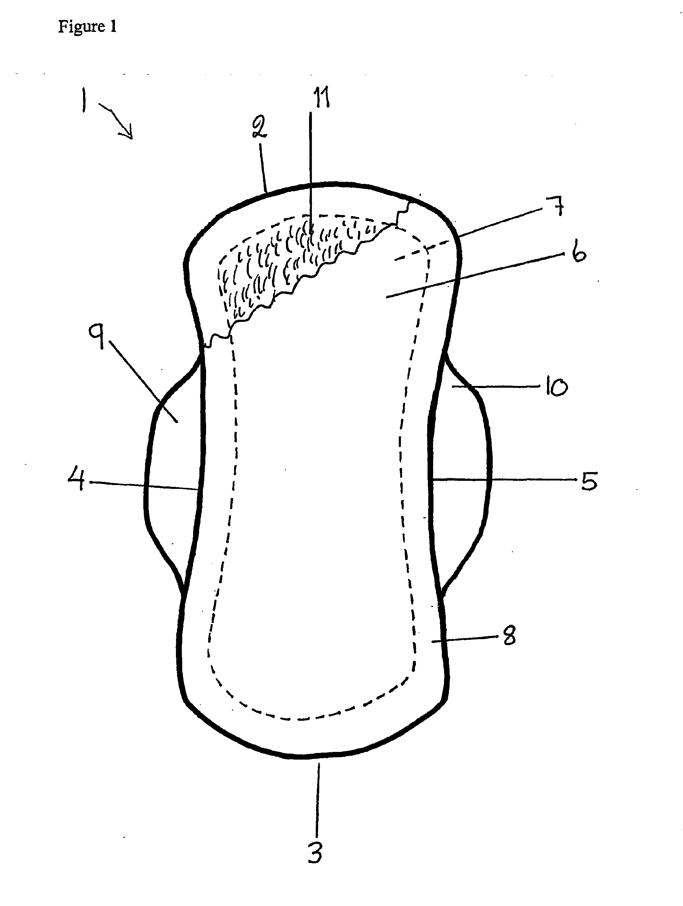

In figure 1 a principal sketch of a sanitary towel 1 according to the invention is shown. However, the invention is not limited to sanitary towels, but may also comprise other absorbent products, such as panty liners, incontinence protections, dressings and tampons, which are intended for absorption of blood or menstrual fluid. In one embodiment the sanitary towel may include two short sides 2, 3 and two long sides 4,5. A liquid permeable surface layer 6 is applied on the side of the sanitary towel 1 facing the wearer during use. The liquid permeable surface layer 6 suitable comprises of a soft, skin-friendly material. Examples of suitable liquid permeable materials are different kinds of non-woven fiber materials, so-called nonwovens. Other usable surface layers are perforated plastic films, nets, knitted, crocheted or woven textiles, and combinations and laminates of the listed material types.

The liquid-blocking backing layer 7 consists of a liquid impermeable material. Thin, liquid-proof plastic films are suitable for the purpose, but it is also possible to use materials which initially are liquid permeable, but which are equipped with a coating of plastics, resin, or some other fluid-proof material. Hereby, the leakage of fluid form the underside of the absorbent product is prevented. The blocking layer 7 may therefore comprise any material meeting the criteria of liquid impermeability, and exhibiting an appropriate sufficient flexibility and skin-friendliness. Examples of materials that are suitable for use as the blocking layer are plastic films, non-woven and laminates of these. The plastic film may for example be of polyethylene, polypropylene or polyester. The blocking layer may alternatively consist of a laminate of a liquid-permeable plastic layer, facing the absorbent body, and a non-woven facing the undergarment of the wearer. A construction of that kind provides a leakage secure layer having a textile character. The fluid-blocking backing layer 7 may also consist of a vapor-permeable material. A breathable backing layer 7 of that kind may for example be a so-called SMS-material (spunbond-meltblown-spunbond) or a breathable plastic film consisting of polyethylene. A plastic film of that kind is disclosed in EP-A-283200. In order to keep the breathability also when the material is applied on a product, the under-side should not be totally covered with fastening means.

The two wrapping layers (the surface layer and the backing layer 6, 7) may be connected to each other and optionally form a protruding connecting edge 8 around the contour line of the sanitary towel. The connection may be accomplished with any technique suitable for the purpose, such as gluing, welding or sewing. The two wrapping layers are not necessary, but may in some cases be suitable.

Between the surface layer and the backing layer 6, 7 a thin flexible absorbent core 11 is placed, which may comprise one or more material layers. The absorbent core 11 is suitably manufactured by one or more layers of cellulose pulp. The pulp may originally be in the form of rolls, bales or sheets, which at the manufacturing of the sanitary towel is dry-defibrated and is transmitted in fluffed form to a pulp mat, sometimes including so-called super-absorbents, which are polymers having the ability to absorb water or body fluids in an amount of several times their own weight. An alternative to this is to dry-form a pulp mat, such as described in WO94/10956. Examples of other usable absorbent materials are different kinds of natural fibers, such as cotton fibers, peat or the like. Naturally, it is also possible to use absorbent synthetic fibers, or particles of a high-absorbing polymer material of the type, which at absorption chemically bind large amounts of liquid, during the formation of a liquid-containing gel, or mixtures of natural fibers or synthetic fibers. The absorbent body 11 may further comprise additional components, such as form-stabilising means, fluid-spreading means, or binders, such as for example thermoplastic fibers, which have been heat-treated to hold short fibers and particles to a connecting unit. It is also possible to use different types of absorbing foam materials in the absorbent body. A further variant is to manufacture the core only of SAP-material.

Preferably, the super-absorbent polymers (SAP) of the invention are manufactured of polyacrylic acid monomers, which are provided from KeboLab, for instance. The monomers are thereafter treated (see example section) in order to form polymers.

They are added to the other absorption material in the form of beads, granules, foam, fibers, threads, film or the like. Granules are preferred in the invention, and they are used at an approximate size of 100 to 850 µm. Other manufacturing methods may of course also be used as long as SAP having the properties above is generated.

Other materials that may be used to produce super-absorbent polymers suitable to use in the invention include hydrolyzed starch-acrylonitrile graft copolymers, starch-acrylic acid graft copolymers, saponified vinyl acetate-acrylic ester copolymers, hydrolyzed acrylonitrile copolymers, hydrolyzed acrylamide copolymers, ethylene-maleic anhydride copolymers, isobutylene-maleic anhydride copolymers, poly-( vinylsulfonic acid ), poly-( vinylphosphonic acid ), poly-( vinylphosphoric acid), poly-( vinylsulfuric acid ), sulfonated polystyrene, poly-( aspartic acid ), poly-( lactic acid ), and mixtures thereof, as well as mixtures of the above mentioned compounds and polyacrylic acid. Naturally, also other material combinations known in the art may be considered, and are thus included in the scope of the invention.

The polymer chains are held together with cross-linkings, which are accomplished by the addition of a cross-linking agent, such as methyl-bisacrylamide (MBA), to the original polymers. Other possible cross-linking agents comprise for example aluminium sulphate, N,N'-methylenebisacrylamide, N,N'-methylenebismethacrylamide, ethylene glycol dimethacrylate, and trimethylolpropane triacrylate. It is to be understood by the skilled person that also other cross-linking agents giving a desired cross-linking are included in the scope of the invention.

According to the invention, the cross-linking agent is added to the SAP-polymer in a concentration of 3 to 7 % mole, preferably 4 to 5 % mole. This has been shown to result in especially advantageous absorption properties for blood or menstrual fluid. These values are measured at a load of 2 kPa, using the AUL-method (Absorption Under Load) in an AUL-cell, which has been modified by exchanging a standard filter to a metal filter (having a mesh size of 100 x 100 µm). These metal filters have enough large meshes for blood cells to pass (about 10 µm in diameter).

The surface of the SAP-particles is treated in a way that it preferably exhibits a certain degree of wettability (hydrophilicity). The wettability is measured by the so-called contact angle. Defibrinated sheep blood is standardly used to measure the contact angle. The contact angle (see for example "Lexikon i KEMI", Gleerups förlag, 1976, 1^{st} edition) specifies the angle between the surface and a water droplet, positioned on the surface. The more hydrophilic surface, the more compressed the droplet becomes (smaller angle). A surface showing a value for the contact angle below 90° is regarded as hydrophilic. In an absorbent product according to the invention a contact angle smaller than 60° is exhibited, preferably smaller than 40°, as measured with defibrinated sheep blood.

In order for the surface of the SAP-particles to exhibit the desired hydrophilicity, the surface is treated with a surface modifier, such as aluminium chloride (AlCl₃) and/or aluminium sulphate (Al₂(SO₄)₃). At treatment of a SAP-particle surface with these ionic compounds, the chloride- and/or sulphate ions will dissociate and a binding of the trivalent, positively charged aluminium ion is obtained on the surface. At absorption of water-containing liquids, the water dipole will then be attracted to and bind to the aluminium ion. Other surface modifiers that may be used include silica compounds, such as silica oxides, and organic compounds, such as ethylene carbonate. All surface modifiers giving the desired effect, i.e. a desired contact angle, are fully possible for use in the invention.

The SAP-particles are added to the absorbent core in an amount of 1 to 100, preferably 10 to 60 % of the total weight of the absorbent core, in order to obtain a good spreading and/or absorption capacity.

The absorbent product of the invention is especially adapted for absorption of blood or menstrual fluid.

Between the surface layer 6 and the absorbent body 11 an inlet layer may be arranged. The purpose of the inlet layer is to direct liquid into the sanitary towel, and to transport it down to the absorbent body 11. The inlet layer may be a non-woven material of low density.

At the downside of the sanitary towel a fastening means may be applied. The fastening means comprises preferably glue, but it may also be a mechanical fastening means such as hook and loop, push buttons, friction linings, clamping means, or the like. The glue may be applied in one or more strings, or in any other pattern. Alternatively, the whole down side of the sanitary towel 1 is lined with glue. It is also possible to use a fastening glue that is breathable and to apply it to the entire downside of the product, for it to function as a combined liquid-proof layer and fastening means. Moreover, the fastening means may be glue towards the body, hydrogels or nothing at all.

On the fastening means, a protection layer may be applied, e.g. when glue is used as the fastening means. The protection layer is preferably a siliconised paper, but also other variants of protection layers are of course possible, such as waxed papers, embossed or release agent-treated plastic film, textile ribbons to fasten to hooks and loops, etc.

The sanitary towel of the invention may further also comprise fastening tabs 9 and 10, which are applied along the long sides of the sanitary towel. The purpose of the tabs is to be folded along the edge of the panty, and thereby keep the sanitary towel in place. Furthermore, other kinds of fastening systems may be used.

Primarily, the absorbent product of the invention is a panty liner, a sanitary towel, or an incontinence protection, adapted for absorption of blood or menstrual fluid.

In yet another aspect, the invention relates to the use of an absorbent product described above for the absorption of blood or menstrual fluid.

The examples below shows preferred embodiments of the SAP-material of the invention, and are not be considered as limiting the scope of the invention in any way.

### Examples

### Example 1

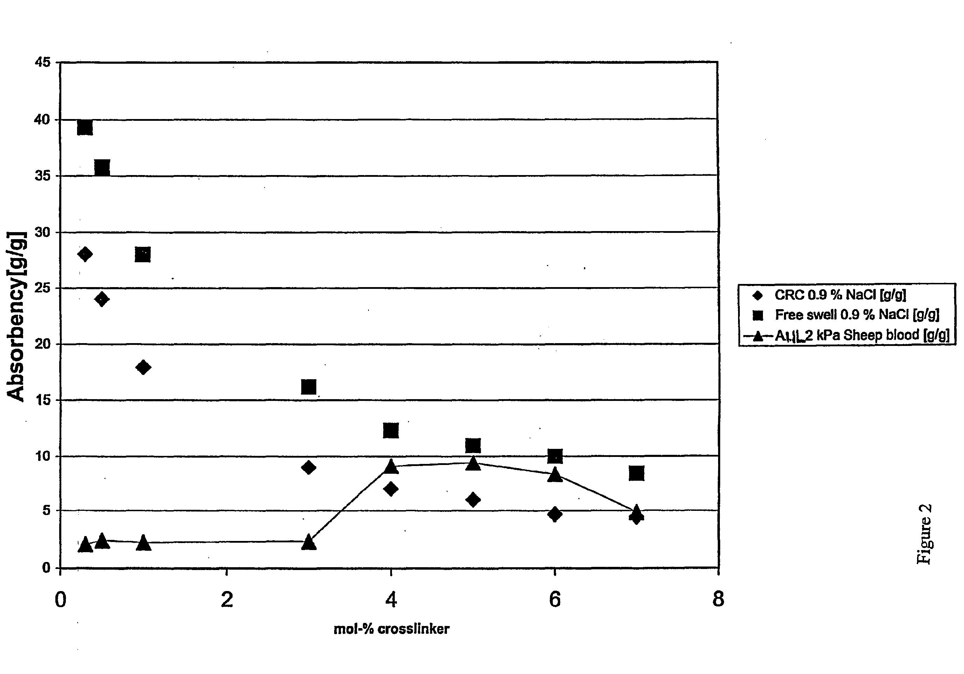

The graph in figure 2 shows the relation between absorption capacity (absorbed weight (g)/ SAP-weight (g)) and the concentration of cross-linking agent (MBA) at different conditions and SAP-material. The first curve (diamonds) shows the absorption capacity (according to edana 441.1-99; centrifuge retention capacity) for a CRC-SAP (cellulose) in 0.9 % NaCl-solution (i.e. a urine like solution). The second curve (squares) shows free absorption capacity in 0.9 % NACl solution for a SAP-gel of polyacrylic acid. The absorption capacity is clearly highest at a low degree of cross-linking and decreases rapidly when the cross-linking degree is increased for both SAP-materials in urine-like conditions. The third curve (triangles) shows the absorption capacity for a polyacrylic acid-SAP in sheep blood at AUL 2 kPa. Here a low absorption is obtained at a low cross-linking degree. However, in the interval from 3 to 7 % mole of cross-linking agent, an increased absorption is obtained. At a cross-linking degree of 5 % mole an absorption capacity of almost 10 g/g in defibrinated sheep blood is obtained.

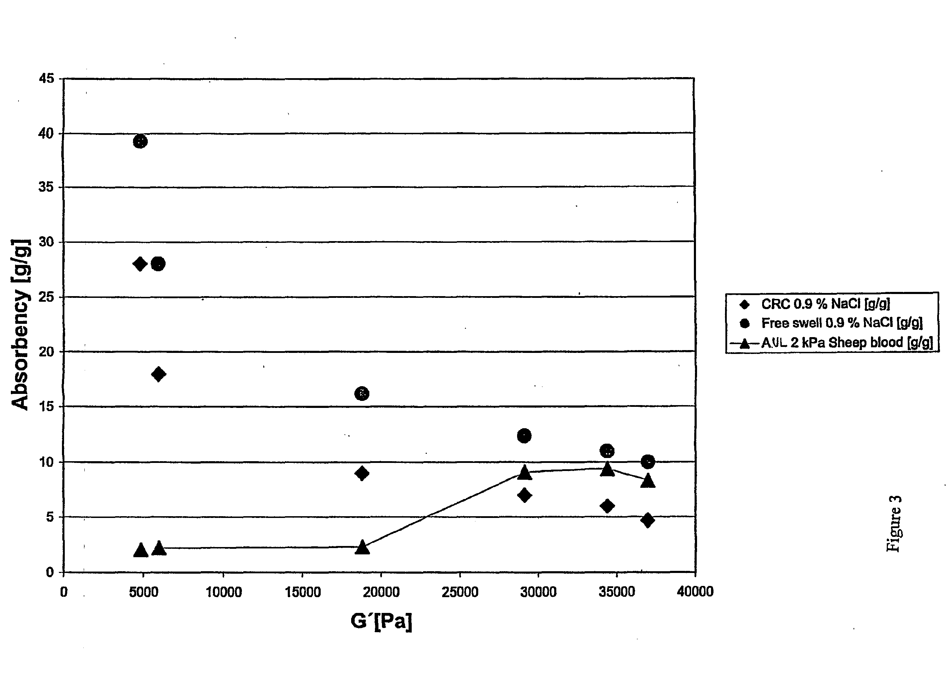

The graph in figure 3 shows the relation between absorption capacity and gel-strength (G' (Pa)). The first curve (diamonds) shows the absorption capacity for a CRC-SAP (cellulose) in 0.9 % NaCl-solution (i.e. a urine-like solution). The second curve (circles) shows free absorption capacity in 0.9 % NaCl-solution for a SAP-gel of polyacrylic acid. The third curve shows the absorption capacity for a polyacrylic acid-SAP in sheep blood at AUL 2 kPa. For curve 1 and 2 the absorption capacity decreases when the gel-strength is increased. For curve 3 a low absorption capacity is obtained with low gel-strength. With a gel-strength above approximately 20000 Pa an increased absorption capacity is achieved, with a top value of between 30000 and 35000 Pa. Here the absorption capacity is almost 10 g/g.

### Example 2 - Manufacture of SAP-particles

To a 25 % acrylic acid-solution, neutralised to 75 %, methylenebisacrylamide is added to a cross-linking degree of 0.1 to 10 %. The reaction is initiated with 0.1 % mole of Va-044 (Wako Pure Chemical Industries LTD, Japan) that is neutralised with sodium ions (such as sodium hydroxide or sodium carbonate). The obtained gel is washed, dryed, grinded (Janke & Kunkel, Analysen Mühce A10) and fractionated to a size of from 100 to 850 µm.

At surface-cross-linking the particles are evaporated in a solution of ethanol, aluminium chloride and sodium hydroxide. This is performed by mixing 25 ml 96 % ethanol, 0.1 g water-free AlCl₃ and 300 µl 4M NaOH with 2 g SAP. The mix is allowed to cross-link for about 30 min at 70 °C, during rotation. Thereafter, the ethanol is removed under vacuum.

### Example 3 - Material properties

The gel-strength of the SAP-gel (shear modules) was determined with a TA instruments AR 1000 N. To 0.5 g SAP-sample 2.5 ml 0.9 % NaCl-solution was added. The elasticity module of the SAP-samples were determined with a TA instruments AR 1000 N. The analyses are performed with a 40 mm acrylic parallel plate, cross hatched at 20 °C. The procedure is oscillating and the samples were analyzed between 0,1 and 100 Hz. The oscillating shear tension is 10.00 Pa. The measured shear tension is G' and was measured at 1 Hz.

The contact angle between defibrinated sheep blood and the SAP-particles was determined with a DAT 1100 Fibro System AB. The analyses were performed in normal laboratory environment at 22 °C and 50 % relative air moisture. For the analyses a particle size of between 100 and 315 µm was chosen.

The resulting contact angle for untreated samples are about 60° with defibrinated sheep blood. After the treatment a contact angle of about 35° is obtained. The AUL-values increases from about 10 g/g to about 13 g/g at a decrease of the contact angle from 60° to 35°.

## Claims

1. An absorbent product (1), such as a panty liner, a sanitary towel, an incontinence protection, a dressing, or a tampon for absorption of blood or menstrual fluid, comprising a fluid permeable surface layer (6) facing the wearer during use, a fluid impermeable backing layer (7) facing away from the wearer during use, and an absorbent body (11) comprising one or more material layers, positioned between the surface layer (6) and the backing layer (7), whereby the absorbent body comprises super-absorbent polymers (SAP), and the SAP-material shows a contact angle at its surface which is smaller than 60°, as measured by defibrinated sheep blood, **characterized by** that the SAP-material shows a gel-strength, which is higher than 18 kPa.

2. Absorbent product according to claim 1, whereby the gel-strength is higher than 25 kPa.

3. Absorbent product according to claim 2, whereby the gel-strength is higher than 30 kPa.

4. Absorbent product according to any one of the preceding claims, whereby the cross-linking degree is in the interval from 3.0 to 7.0 % mole.

5. Absorbent product according to claim 4, whereby the cross-linking degree is in the interval from 4.0 to 5.0 % mole.

6. Absorbent product according to any one of the preceding claims, whereby the product shows an absorption capacity of more than 6 g blood/g SAP under load.

7. Absorbent product according to claim 6, whereby the absorption capacity is at least 10 g blood/g SAP under load.

8. Absorbent product according to claim 7, whereby the absorption capacity is at least 13 g blood/g SAP under load.

9. Absorbent product according to any one of the preceding claims, whereby the contact angle is smaller than 40°.

10. Absorbent product according to any one of the preceding claims, whereby the amount of SAP in the absorbent body is in the interval from 1 to 100 % weight.

11. Absorbent product according to claim 10, whereby the amount of SAP is in the interval from 10-60 % weight.

12. Absorbent product according to any one of the preceding claims, wherein the SAP-material is manufactured, partly or completely, from polyacrylic acid.

13. Absorbent product according to any one of the preceding claims, wherein the surface of the SAP-particles has been treated with aluminium chloride and/or aluminium sulphate.

14. Use of an absorbent product according to any one of the preceding claims for the absorption of blood or menstrual fluid.

## Patentansprüche

1. Absorbierendes Produkt (1), wie beispielsweise eine Slipeinlage, eine Binde, ein Inkontinenzschutz, ein Verband, oder ein Tampon zur Absorption von Blut oder Menstrualfluid, umfassend eine flüssigkeitsdurchlässige Oberflächenlage (6), die im Gebrauch dem Träger zugewandt ist, eine flüssigkeitsundurchlässige Decklage (7), die im Gebrauch vom Träger weg weist, und einen Absorptionskörper (11) aus einer oder mehreren Materiallagen, der zwischen der Oberflächenlage (6) und der Decklage (7) positioniert ist, wobei der Absorptionskörper ein superabsorbierendes Polymer (SAP) umfasst und das SAP-Material an seiner Oberfläche einen Berührungswinkel aufweist, der gemessen mit defibriniertem Schafsblut kleiner als 60° ist, **dadurch gekennzeichnet, dass** das SAP-Material eine Gelfestigkeit aufweist, die größer als 18 kPa ist.

2. Absorbierendes Produkt nach Anspruch 1, bei dem die Gelfestigkeit größer als 25 kPa ist.

3. Absorbierendes Produkt nach Anspruch 2, bei dem die Gelfestigkeit größer als 30 kPa ist.

4. Absorbierendes Produkt nach einem der vorstehenden Ansprüche, bei dem der Vernetzungsgrad im Bereich von 3,0 bis 7,0% Mol liegt.

5. Absorbierendes Produkt nach Anspruch 4, bei dem der Vernetzungsgrad im Bereich von 4,0 bis 5,0% Mol liegt.

6. Absorbierendes Produkt nach einem der vorstehenden Ansprüche, bei dem das Produkt unter Last eine Absorptionsfähigkeit von mehr als 6 g Blut/g SAP aufweist.

7. Absorbierendes Produkt nach Anspruch 6, bei dem die Absorptionsfähigkeit unter Last wenigstens 10 g Blut/g SAP beträgt.

8. Absorbierendes Produkt nach Anspruch 7, bei dem die Absorptionsfähigkeit unter Last wenigstens 13 g Blut/g SAP beträgt.

9. Absorbierendes Produkt nach einem der vorstehenden Ansprüche, bei dem der Berührungswinkel kleiner als 40° ist.

10. Absorbierendes Produkt nach einem der vorstehenden Ansprüche, bei dem die Menge an SAP im Absorptionskörper in einem Bereich von 1 bis 100 Gewichts-% liegt.

11. Absorbierendes Produkt nach Anspruch 10, bei dem die Menge an SAP in einem Bereich von 10 - 60 Gew.-% liegt.

12. Absorbierendes Produkt nach einem der vorstehenden Ansprüche, bei dem das SAP-Material teilweise oder vollständig aus Polyacrylsäure hergestellt ist.

13. Absorbierendes Produkt nach einem der vorstehenden Ansprüche, bei dem die Oberfläche der SAP-Partikel mit Aluminiumchlorid und/oder Aluminiumsulfat behandelt wurde.

14. Verwendung eines absorbierenden Produkts gemäß einem der vorstehenden Ansprüche zur Absorption von Blut oder Menstrualfluid.

## Revendications

1. Article absorbant (1), tel un protège-slip, une serviette hygiénique, une protection contre l'incontinence, un pansement ou un tampon conçu pour absorber du sang ou du liquide menstruel, comprenant une couche superficielle (6) perméable aux fluides, placée du côté du porteur en cours d'usage, une couche d'envers (7) imperméable aux fluides, placée du côté opposé à celui du porteur en cours d'usage, et un corps absorbant (11) comprenant une ou plusieurs couche(s) de matériau, disposé entre la couche superficielle (6) et la couche d'envers (7), lequel corps absorbant comprend des polymères super-absorbants (PSA), la matière PSA présentant un angle de contact à sa surface, mesuré avec du sang de mouton défibriné, inférieur à 60°, **caractérisé en ce que** cette matière PSA présente, à l'état de gel, une résistance mécanique supérieure à 18 kPa.

2. Article absorbant conforme à la revendication 1, dans lequel ladite résistance mécanique à l'état de gel est supérieure à 25 kPa.

3. Article absorbant conforme à la revendication 2, dans lequel ladite résistance mécanique à l'état de gel est supérieure à 30 kPa.

4. Article absorbant conforme à l'une des revendications précédentes, dans lequel le degré de réticulation se situe dans l'intervalle allant de 3,0 à 7,0 % en moles.

5. Article absorbant conforme à la revendication 4, dans lequel le degré de réticulation se situe dans l'intervalle allant de 4,0 à 5,0 % en moles.

6. Article absorbant conforme à l'une des revendications précédentes, lequel article présente une capacité d'absorption de plus de 6 g de sang par gramme de PSA, sous charge.

7. Article absorbant conforme à la revendication 6, dans lequel ladite capacité d'absorption vaut au moins 10 g de sang par gramme de PSA, sous charge.

8. Article absorbant conforme à la revendication 7, dans lequel ladite capacité d'absorption vaut au moins 13 g de sang par gramme de PSA, sous charge.

9. Article absorbant conforme à l'une des revendications précédentes, dans lequel ledit angle de contact est inférieur à 40°.

10. Article absorbant conforme à l'une des revendications précédentes, dans lequel la proportion pondérale de PSA dans le corps absorbant se situe dans l'intervalle allant de 1 à 100 %.

11. Article absorbant conforme à la revendication 10, dans lequel ladite proportion pondérale de PSA se situe dans l'intervalle allant de 10 à 60 %.

12. Article absorbant conforme à l'une des revendications précédentes, dans lequel la matière PSA est faite, en partie ou en totalité, de poly(acide acrylique).

13. Article absorbant conforme à l'une des revendications précédentes, dans lequel les particules de PSA ont subi un traitement de surface effectué avec du chlorure d'aluminium et/ou du sulfate d'aluminium.

14. Emploi d'un article absorbant, conforme à l'une des revendications précédentes, pour l'absorption de sang ou de liquide menstruel.
